# EUROPEAN PATENT SPECIFICATION

(11) **EP 3 096 702 B1**
(45) Date of publication and mention of the grant of the patent: **19.06.2019**
(21) Application number: 15700014.2
(22) Date of filing: 04.01.2015
(51) Int. Cl.: A61B 18/20, A61B 18/00

(54) **DEVICE AND METHOD FOR NON-INVASIVE TREATMENT OF SKIN USING LASER LIGHT**
VORRICHTUNG UND VERFAHREN ZUR NICHTINVASIVEN BEHANDLUNG DER HAUT MIT LASERLICHT
DISPOSITIF ET PROCÉDÉ DE TRAITEMENT NON INVASIF DE LA PEAU À L'AIDE D'UNE LUMIÈRE LASER

(30) Priority: 21.01.2014 EP 14151850
(43) Date of publication of application: 30.11.2016
(73) Proprietor: Koninklijke Philips N.V., 5656 AE Eindhoven (NL)
(72) Inventor: VARGHESE, Babu, 5656 AE Eindhoven (NL); JURNA, Martin, 5656 AE Eindhoven (NL); PALERO, Jonathan Alambra, 5656 AE Eindhoven (NL); HORTON, Margaret Ruth, 5656 AE Eindhoven (NL); ZEITOUNY, Mounir, 5656 AE Eindhoven (NL); VERHAGEN, Rieko, 5656 AE Eindhoven (NL)
(74) Representative: Wolfs, Marc Johannes Maria
(86) International application number: PCT/EP2015/050018
(87) International publication number: WO 2015/110273

(56) References cited:
- WO-A2-2005/037234
- WO-A2-2005/122694
- WO-A2-2008/001284
- US-A- 5 786 924

## Description

### FIELD OF THE INVENTION

The invention relates generally to the treatment of skin using laser light, and more particularly to a non-invasive device and method for performing said treatment.

### BACKGROUND OF THE INVENTION

Various forms of electromagnetic radiation, particularly laser light beams, have been used on the skin for many years for a variety of treatments, such as hair removal, skin rejuvenation to reduce wrinkles and reduction of pigmentation spots, and the treatment of conditions like acne, actinic keratosis, blemishes, scar tissue, discoloration, vascular lesions, acne treatment, cellulite and tattoo removal. Most of these treatments rely on photothermolysis, where a treatment location is targeted by the treatment radiation. Preferably, the treatment radiation is configured to be mainly absorbed at the treatment location, such that the temperature at the treatment location is raised sufficiently to achieve the desired thermal damage, for example tissue necrosis, denaturation or coagulation.

In skin rejuvenation treatments, the treatment laser beam passes through the outer layers of the skin to the dermis layer. The dermis is damaged by heating to induce a wound response, without damage to the epidermis. In other words, a target area within the skin is damaged in a controlled way, and the skin is allowed to replace the damaged tissue by new collagen growth - the damage promotes healing and a rejuvenation effect occurs. The renewed tissue improves the skin's radiance, tone, and can even provide a reduction in pore size, wrinkles and fine lines.

Selective non-ablative photothermolysis based on water absorption is used to heat the tissue to between 60 and 100 degrees Celsius to induce damage in the selected areas, without ablation or vaporization of the skin. Ablative photothermolysis occurs when the water temperature exceeds 100 degrees Celsius.

Focused laser light may even be used to create laser induced optical breakdown (LIOB) inside the dermis layer, as known from the published international patent application WO 2008/001284 A2.

Since many skin treatments require a treatment location in an inner skin layer, such as the dermis, damage to an adjacent outer layer, such as the epidermis, is a major problem. Subjecting the epidermis to high intensities may cause undesirable heating, which may result in additional discomfort to the subject being treated, adverse skin reactions and unwanted changes in skin pigment. This is particularly undesirable when treating facial disorders such as rhytides and wrinkles.

Techniques to reduce heating of the epidermis are known, for example, from the article "Spatially confined photothermolysis of dermal targets using an IR-fiber laser in combination with focusing and contact cooling," Lasers Surg Med., 14 (Suppl):28 (2002) by Manstein D, Poureshagh M, Yaroslavsky I, Altshuler GB, and Anderson RR. Here, active surface cooling was applied in parallel to avoid epidermal injury when treating the skin by intradermal focusing of infrared fiber (λ=1.06 and 1.2 µm) laser pulses. However, active surface cooling makes the treatment device more complicated and expensive. This is particularly disadvantageous for home-use applications.

A different approach was attempted in PCT application WO 2005/122694 by spreading out the radiation by means of a beam conversion system between the radiation source and the skin surface. The beam conversion system deflects the incident radiation beam off-axis, and then redirects back at an angle to cross the symmetry axis at the target point under the skin. Two main embodiments are described - one in which a single beam is rotated around the target axis at a fixed angle, and the other where the radiation beam is split into a plurality of angled beams. The beam conversions system makes this system very complex and difficult to adjust.

A further known technique is to separate the laser pulses by a pulse delay of sufficient length to provide normal thermal relaxations for the epidermis. This is described in the article "Intense Pulsed Light as a Nonablative Approach to Photoaging", Dermatol Surg 2005; 31:1179-1187, by Goldman, Weiss R, and Weiss M. In particular, on pages 1180-1181, a pulse delay of at least 10 milliseconds is recommended in general, and for some subjects 20 to 30 milliseconds is recommended.

As the treatment efficacy depends on both the temperature at the target location and the duration of the treatment, the use of excessively long pulse delays may increase the overall duration of the treatment.

It is therefore desirable to improve the efficacy of such treatments, while keeping the treatment device as simple as possible.

### SUMMARY OF THE INVENTION

The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes. An object of the invention is to provide a non-invasive skin treatment device and method for treatment of an inner skin layer using laser light.

The object is achieved according to the invention by a device comprising:
- a light source and an associated optical system for generating pulses of a laser treatment beam) along a treatment optical axis of the device, the light source and optical system being configured and arranged to provide, within a transverse cross-section of the laser treatment beam perpendicular to the treatment optical axis:
   - a first and a second beam region of non-zero light intensity disposed at a periphery of the transverse cross-section, and
   - a third beam region, arranged between the first and the second beam region, of lower light intensity than said non-zero light intensity;
   the optical system being further configured and arranged:
   - to focus, in use, the treatment laser beam to a focal spot in the inner skin layer disposed on the treatment optical axis;
   - to guide, in use, the laser treatment beam such that the laser treatment beam with the first and the second beam region passes along the treatment optical axis through an outer skin layer to the focal spot, whereby the first and the second beam region pass through, respectively, a first and a second skin region in the outer skin layer;
wherein an extent of the third beam region is predetermined and/or controlled to provide a corresponding third skin region in the outer skin layer, arranged between the first and the second skin region,
wherein the lower light intensity of the third beam region is predetermined and/or controlled to provide a maximum temperature during a pulse of the laser treatment beam within the third skin region which is lower than maximum temperatures during said pulse of the laser treatment beam within the first and the second skin region;
and wherein the light source is configured and arranged to provide, in use, a pulse duration of the pulse of the laser treatment beam which is longer than a thermal relaxation time of the first and the second skin region.

The object is also achieved by a non-invasive method of treating an inner skin layer using a device for generating laser light, the device comprising a light source and an associated optical system for generating pulses of a laser treatment beam along a treatment optical axis of the device,
the method comprising:
- generating the laser treatment beam with a transverse cross-section perpendicular to the treatment optical axis comprising:
   - a first and a second beam region of non-zero light intensity disposed at a periphery of the transverse cross-section, and
   - a third beam region, arranged between the first and the second beam region, of lower light intensity than said non-zero light intensity;
   - focusing the treatment laser beam to a focal spot in the inner skin layer, disposed on the treatment optical axis, using the optical system;
   - guiding the laser treatment beam such that the laser treatment beam with the first and the second beam region passes through an outer skin layer along the treatment optical axis to the focal spot, whereby the first and the second beam region pass through, respectively, a first and a second skin region in the outer skin layer;
- predetermining and/or controlling an extent of the third beam region to provide a corresponding third skin region in the outer skin layer, arranged between the first and the second skin region,
- predetermining and/or controlling the lower light intensity of the third beam region to provide a maximum temperature during a pulse of the laser treatment beam within the third skin region which is lower than maximum temperatures during said pulse of the laser treatment beam within the first and the second skin region; and
- configuring and arranging the light source to provide, in use, a pulse duration of the pulse of the laser treatment beam which is longer than a thermal relaxation time of the first and the second skin region.

The invention is based on the insight that providing at least one central beam region of significantly lower laser intensity, within the transverse cross-section of the laser treatment beam, provides a corresponding central skin region in an outer skin layer. The lower laser intensity in this central skin region may be configured and arranged to provide a lower degree of heating than in the surrounding skin regions. In other words, a heat-sink region is provided for the surrounding skin regions. This is advantageous because the heating of the outer layer of skin may be more uniformly distributed than when use is made of a beam with a conventional Gaussian or top-hat intensity profile, thus avoiding hot spots which can result in undesired thermal damage in the outer skin layer. Controlling and/or predetermining the extent of this heat-sink region are convenient ways of preventing excessive temperatures in the outer skin layer.

The light source is configured and arranged to provide, in use, a pulse duration of the pulse of the laser treatment beam which is longer than the thermal relaxation time of the first and the second skin region. The invention thus makes it possible to treat an inner skin layer using longer duration pulses, without using further measures such as external cooling. The inner heat-sink region in the outer skin layer provides a skin region within which excess heat from the surrounding skin regions may dissipate.

It may be also advantageous for the light source or optical system to further comprise at least one optical element configured and arranged to provide the first, the second and the third beam region. This optical element (or optical elements) may be disposed within the light source, for example in the laser cavity or in the optical system. Any suitable optical element known in the art may be used, for example, a central mask, a ring aperture, a Spatial Light Modulator (SLM), a Diffractive-Optical element (DOE), a phase mask, a spiral wave plate, a vortex wave plate, a Pitch-Fork Hologram, a Q-Plate, or a Cylindrical Mode Converter. It may be advantageous to combine more than one of these optical elements to provide the beam regions. It may also be advantageous to provide at least one optical element inside the light source, in combination with at least one optical element in the optical system.

Although the invention may provide many configurations of the beam regions, it may be particularly advantageous to provide a first and a second beam region that are non-contiguous, and to configure the third beam region so as to separate the first and the second beam region throughout the length of their borders within the transverse cross-section of the laser treatment beam.

In a further advantageous configuration, the first and the second beam region are contiguous and form part of an annular region of non-zero light intensity, the third beam region being configured to form a central region of lower light intensity, and wherein the central region of lower light intensity is enclosed by the annular region of non-zero light intensity. Such a ring-shaped or doughnut-shaped pattern may be provided using an appropriately placed central aperture, or many other techniques known to the skilled person.

It may be advantageous to configure and arrange the light source to provide, in use, a pulse duration of the pulse of the laser treatment beam which is longer than a thermal relaxation time of the outer skin layer and shorter than a thermal relaxation time of the inner skin layer. For example, if the outer skin layer is the epidermis, the pulse duration should be longer than or equal to 10 to 30 ms. These values are typical values of the thermal relaxation time of the epidermis. If the inner skin layer is the dermis, the pulse duration should be shorter than or equal to 100ms to 300 ms. These values are typical values of the thermal relaxation time of the dermis. Such pulse durations are expected to effectively heat the dermis in focused positions, while sparing the epidermis from unnecessary thermal damage. In particular, the pulse duration may be predetermined and/or controlled to be longer than the thermal relaxation time of the skin tissue present in the first and the second skin region and shorter than the thermal relaxation time of the skin tissue or chromophore present at the focal spot of the laser treatment beam.

### BRIEF DESCRIPTION OF THE DRAWINGS

Fig. 1 diagrammatically shows the most important parts of the device 10 in use when treating an inner layer 17 of skin 15,
Fig. 2 schematically depicts the beam regions 22a, 22b of non-zero intensity in the treatment laser beam 22,
Fig. 3 and Fig. 4 show examples of the transverse cross-section of the laser treatment beam 22, 122, comprising beam regions of non-zero intensity 22a, 122a and 22b, 122b, separated by a beam region of lower intensity 22c, 122c,
Fig. 5A depicts schematically the intensity across the beam profile for a Gaussian and a flat-top laser treatment beam 22,
Fig. 5B depicts schematically the temperature profile in the epidermis resulting from treatment of an inner skin layer using the laser treatment beams depicted in figure 5A,
Fig. 6A depicts schematically the intensity across the beam profiles for a modified Gaussian and a modified flat-top laser treatment beam 22 according to the invention,
Fig. 6B depicts schematically the temperature profile in the epidermis resulting from treatment of an inner skin layer using laser treatment beams depicted in figure 6A,
Fig. 7 depicts the results of a simulation to determine the relationship 70 between thermal penetration depth and pulse duration for an outer skin layer,
Fig. 8 depicts examples of known rectangular transverse laser modes that may be provided by the correspondingly configured device, for example, Hermite-Gaussian modes, and
Fig. 9 depicts examples of known cylindrical transverse laser modes that may be provided by the correspondingly configured device, for example, Laguerre-Gaussian modes.

It should be noted that items which have the same reference numbers in different Figures, have the same structural features and the same functions, or are the same items. Where the function and/or structure of such an item have been explained, there is no necessity for repeated explanation thereof in the detailed description.

### DETAILED DESCRIPTION OF EXAMPLES

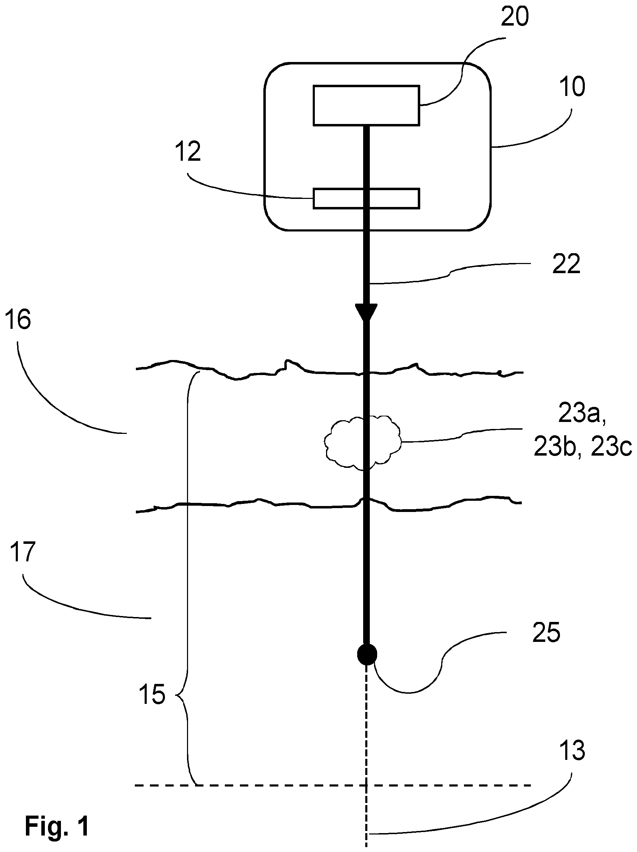

Figure 1 schematically shows a laser treatment device 10 according to the invention, comprising a light source 20 and an optical system 12, configured and arranged for generating a laser treatment beam 22 along a treatment optical axis 13 of the device 10. The laser light beam 22 is of a suitable frequency and pulse duration for treating an inner layer 17 of human or animal skin 15. The light source 20 is typically a pulsed laser, for example, a diode laser with emission at 1435 nm and 1-1000 ms pulse duration. During use, the laser light exits the device 10 as a treatment laser beam 22 along the treatment optical axis 13 which impinges on the outer surface of the skin 15. The device 10 is arranged to treat an inner layer of the skin, such as the dermis 17, wherein the treatment laser beam 22 passes through an outer layer 16 of the skin 15 along the treatment optical axis 13. The optical system 12 is configured and arranged to focus the treatment beam 22 to a focal spot 25 in the inner skin layer 17. The focal spot 25 is disposed on the treatment optical axis 13.

Focusing the beam increases the laser intensity in the focal spot 25, which increases the efficacy of the treatment because the focal spot 25 will reach higher temperatures than the outer skin layer 16. This provides a relatively simple focusing system, allowing a dynamic control of the focusing depth. This is different to known devices, such as those described in WO 2005/122694 which use very complicated beam conversion systems to split the incident beam into multiple beams, to deflect tham aways from the axis, and to redirect them to intersect below the surface of the skin.

The focal spot 25 is selected to correspond to the treatment location - in most applications, the focal spot 25 will be disposed at the treatment location.

In some applications, it may also be advantageous to dispose the focal spot 25 proximate to the treatment location, whereby the heat from the focal spot 25 diffuses towards the treatment location. The focal spot is then selected to coincide with a suitable chromophore in the inner skin layer 17.

The light source 20 and optical system 12 may also be configured to provide a more diffuse treatment beam 22 by not focusing the treatment beam 22. In that case, the treatment location in the inner skin layer 17 will be determined by suitably located chromophores which are irradiated by the treatment beam 22. The optical system 12 is suitably configured to guide the first 22a and the second 22b beam region through an outer skin layer 16 to the treatment location, whereby the first 22a and the second 22b beam region pass through, respectively, a first 23a and a second 23b skin region in the outer skin layer 16.

The skin 15 comprises multiple layers with different optical properties. The epidermis 16 is composed of the outermost skin layers and forms a waterproof protective barrier. The outermost layer of the epidermis 16 is the stratum corneum. The dermis 17 is situated underneath the epidermis 16.

According to the preferred use of the invention, the focal spot 25 is disposed in the dermis 17, and the first 23a and the second 23b skin region are present in the stratum corneum, epidermis 16, or skin surface layer. The extent of the third beam region 23c is predetermined and/or controlled to provide the required extent of the corresponding third skin region. The extent of the third skin region 23c is selected to provide a heat sink of suitable dimensions to ensure that the maximum temperature during the treatment within the third skin region 23c in the outer skin layer 16 is lower than the maximum temperatures in the surrounding regions in the first 23a and the second 23b skin region.

The temperature within the skin regions may be monitored during use, using any convenient apparatus known in the art, such as an infrared camera or a microphone. Alternatively or additionally, skin simulation may be used to configure and arrange the device of the invention such that the maximum temperatures are not exceeded.

If the device 10 is used to reduce wrinkles in the skin 15, the focal spot 25 is disposed in the collagen of the dermis 17 in order to create microscopic lesions at the position of the focal spot 25, which results in new collagen formation. The focal spot 25 may then be disposed between 0.2 and 2 mm below the outer surface of the skin 15, in particular between 0.5 and 1.5 mm below the outer surface of the skin 15. Typically, the lesions formed will be larger than the volume of the focal spot 25.

The optical system 12 is configured and arranged to guide, in use, the laser treatment beam 22 to exit the device 10 along the treatment optical axis 13 which then impinges on an outer surface of the skin 15 to be treated, and to guide, in use, the treatment laser beam 22 to the focal spot 25 in an inner skin layer 17 along the treatment optical axis 13. The word "guide" includes configurations where the direction of the light beams is changed substantially, and configurations where the treatment beam 22 is allowed to propagate along the light beam direction without substantial change, and any intermediate degree of directional change. In all cases, the treatment beam 22 will exit the device 10 along the treatment optical axis 13.

The laser treatment beam 22 that impinges on an outer surface of the skin 15 will be approximately the same diameter as the beam that was produced by the light source 20. This is different to known devices, such as those described in WO 2005/122694 which use a beam conversion system to create a spot on the surface of the skin which has a total area larger than the cross-section area of the input light beam.

Consequently, the treatment light beam 22 will pass through an outer skin layer on its way to the focal spot 25. The device 10 according to the invention is configured and arranged to prevent or mitigate thermal damage in this outer skin layer during treatment at a treatment location corresponding to the focal spot 25. In other words, the invention protects the outer skin layer 16 from hot spots.

The phrase "outer skin layer" should be interpreted to include more than just the epidermis 16 - the invention may also be used to protect the surface of the skin 15, or even the upper layer of the dermis 17. The phrase "inner skin layer" should be interpreted to include more than just the dermis 17 - the focal spot 25 may be disposed in an upper layer of the dermis 17, or even in the epidermis 16. The only restriction is that the outer skin layer is located between the focal spot 23, disposed in the inner skin layer, and the device 10.

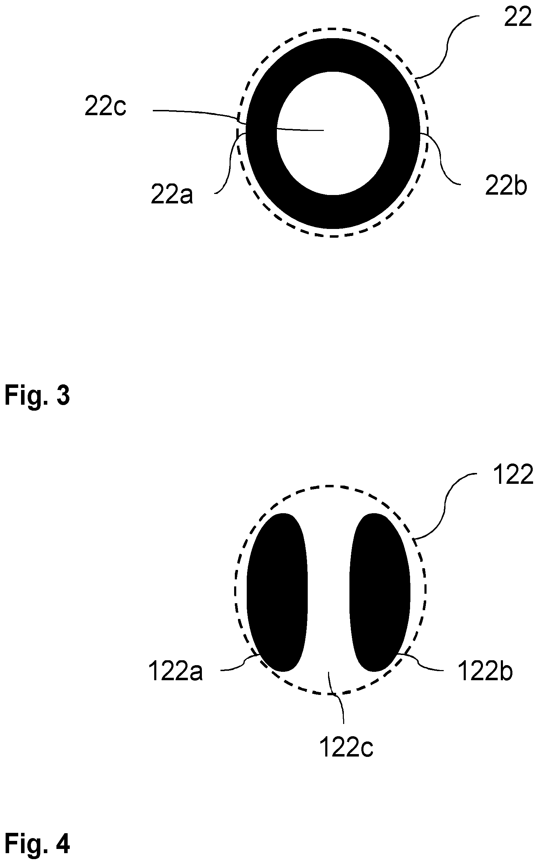

Figure 3 depicts a transverse cross-section through the laser treatment beam 22. A ring-shaped outer beam region 22a, 22b of non-zero light intensity is provided, comprising an inner disc-shaped beam region 22c having a substantially lower light intensity than the outer ring 22a, 22b. Typically, the inner disc-shaped beam region 22c will be approximately 100-200 microns in diameter, compared to an outer ring 22a, 22b diameter of approximately 200-500 microns. The edge of each beam region 22a, 22b may be considered to be a contour joining points whose intensity is equal to half the maximum intensity within the beam region.

This "doughnut" cross-section may be provided by the light source 20 or optical system 12 comprising at least one optical element which reduces, or even blocks, the light intensity at the inner beam region 22c of the treatment beam 22. Any suitable optical elements known in the art may be used, for example, a central mask, a ring aperture, a Spatial Light Modulator (SLM) as known from US patent 7961371, a Diffractive-Optical element (DOE), a phase mask as known from US patent 7982938, a spiral waveplate, a vortex waveplate, a Pitch-Fork Hologram, a Q-Plate, or a cylindrical Mode Converter. A plurality and/or a combination of such elements may also be used.

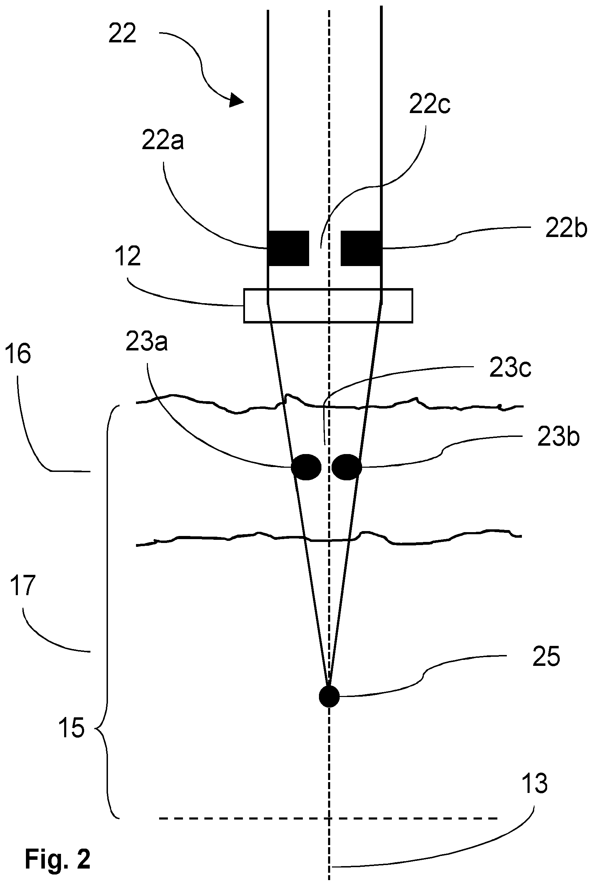

Figure 2 depicts schematically a longitudinal cross-section through the laser treatment beam 22, provided by the device 10 depicted in Figure 1, which laser treatment beam is configured and arranged to provide the transverse cross-section depicted in Figure 3.

Figure 2 further depicts the laser treatment beam 22, having a central region 22c of low intensity, the laser treatment beam impinging on the outer surface of the skin 15 and passing through an outer skin layer 16 to the focal spot 25. The outer skin layer is located in the epidermis 16, and the focal spot 25 is disposed in the dermis 17. The optical system 12 is configured to focus the treatment beam 22 to a focal spot at or proximate to the treatment location.

In longitudinal cross-section, the laser treatment beam comprises a first 22a and a second 22b beam region of non-zero light intensity disposed at the periphery of the transverse cross-section, and a third beam region 22c, extending between the first 22a and the second 22b beam region, and being of substantially lower light intensity than the non-zero light intensity. When viewed in transverse cross-section, the first 22a and the second 22b beam region are portions of the ring-like outer beam region depicted in Figure 3.

As the laser treatment beam 22 passes through the outer skin layer 16, it creates a first 23a and a second skin region 23b of non-zero light intensity in the outer skin layer 16, corresponding to the first 22a and the second 22b beam region of non-zero intensity in the treatment beam 22. The relationship between the dimensions and intensities of the beam regions in the treatment beam 22 and the skin regions 23a, 23b in the outer skin layer 16 depends on the scattering and absorption properties of the outer skin layer 16, and on the tissues located between the first 23a and second 23b skin region and the outer surface of the skin 15. The dimensions and intensities in the skin regions 23a, 23b of the outer skin layer 16 determine the temperature rise in those skin regions 23a, 23b.

The first 22a and the second 22b non-zero intensity beam region are separated by a third beam region 22c having a lower light intensity than the non-zero light intensity. This lower light intensity is substantially lower than the non-zero intensity. This creates a third skin region 23c in the outer skin layer having a substantially lower intensity than the first 23a and the second 23b skin region. Again, the relationship between the dimensions and intensities of the central beam region 22c and the central skin region 23c depends on the scattering and absorption properties of the outer skin layer, and on the tissues located between the central skin region 23c and the outer surface of the skin 15. Although this central skin region 23c may have its temperature raised by the intensity of the laser light in this skin region 23c, the extent of the third beam region 22c is predetermined and/or controlled to provide a suitably-dimensioned heat sink between the first 23a and the second 23b skin region.

The intensity profile of the laser treatment beam 22 at the treatment location may also comprise non-zero intensity and lower-intensity regions. But the dimensions and intensities of such regions at the treatment location depend on the degree of focusing used, and the scattering and absorption properties of the treatment location, and on the tissues located between the treatment location and the outer surface of the skin 15.

If the treatment beam 22 is focused to a focal spot 25 in the dermis 17, the regions of significantly different intensity will no longer be preserved due to thermal diffusion. The focusing helps localize the region being heated. It also helps prevent damage to the epidermis 16 because the power density may be much lower in the epidermis 16 than in the dermis 17.

For the invention it is not necessary that a further heat sink skin region is created at the treatment location.

During treatment at the treatment location, the temperature will rise in the first 23a and the second 23b skin region of the outer skin layer 16. By providing a heat sink between these skin regions, a higher degree of thermal diffusion and thermal redistribution means that the risk of thermal damage within the first 23a and the second 23b skin region is reduced. By appropriate configuration of the device 10, the heat-sink dimensions may be determined such that, during the treatment, the maximum temperature within the third central skin region 23c is lower than the maximum temperatures in the first 23a and the second 23b skin region.

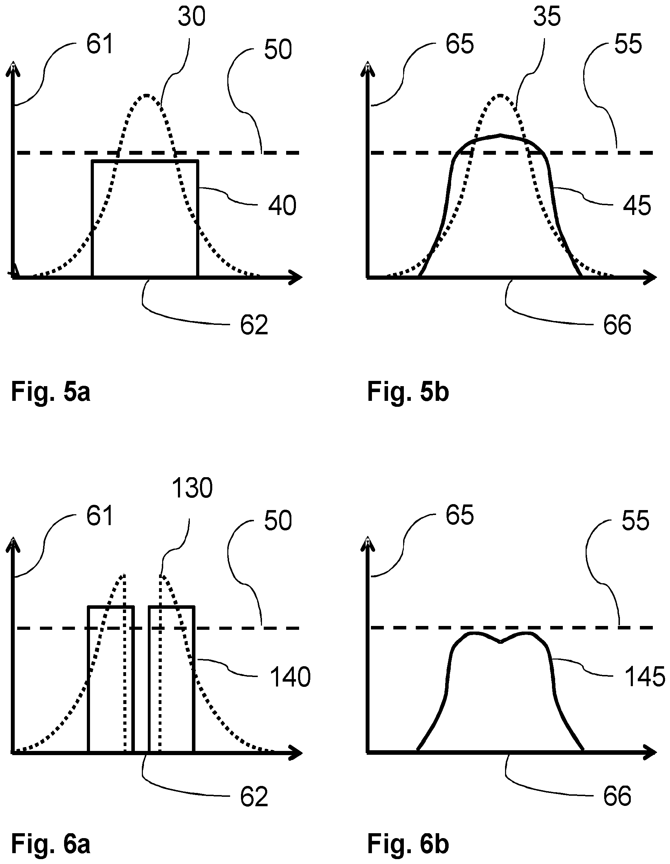

The principle of operation may also be understood from the graphs provided as figures 5a, 5b, 6b and 6c. Figure 5a depicts two laser beam intensity profiles known in the art - a conventional Gaussian profile 30 and a conventional flat-top profile 40. The vertical axis 61 represents intensity of the laser light as power per unit area, and the horizontal axis 62 represents the position through a transverse cross-section of the laser treatment beam 22. The laser intensity within the flat-top profile 40 is approximately constant across the diameter of the beam cross-section. An intensity threshold 50 is also depicted, parallel to the horizontal axis, at an intensity approximately equal to the maximum intensity of the flat-top profile 40. The intensity in the Gaussian profile 30 shows a Gaussian distribution across the beam cross-section, with a central maximum significantly exceeding the intensity threshold 50. The Gaussian profile 30 and flat-top profile 40 represent beams having the same power.

When the laser beam profiles are used to treat skin 15, the epidermal 16 temperature profiles depicted in Figure 5b result. The vertical axis 65 represents the temperature in the epidermis after exposure to a plurality of laser pulses, and the horizontal axis 66 represents the position through a transverse cross-section of the outer skin location where the maximum temperature is to be controlled. A Gaussian temperature profile 35 and a flat-top temperature profile 45 are depicted, resulting from exposure to, respectively, the Gaussian beam intensity profile 30 and the flat-top intensity profile 40. A temperature threshold 55 is also depicted, parallel to the horizontal axis at a temperature approximately equal to the maximum temperature desired in the epidermis. The Gaussian temperature profile 35 shows a Gaussian distribution across the outer skin location 23, with a central maximum significantly exceeding the temperature threshold 55. This "hot spot" is due to the peak intensity in the intensity profile 35, and is a known problem in the art.

Conventionally, this "hot spot" is avoided by using the flat-top intensity profile 40. However, the associated temperature profile is not a flat-top. Figure 5b depicts the respective flat-top temperature profile 45 across the outer skin location, which resembles a bell shape. The central portion of the profile 45 extends beyond the temperature threshold 55, representing a "hot spot" in the epidermis. The assumption that the flat-top intensity profile 40 produces a flat-top temperature profile is true for a relatively short treatment time or relatively short pulses. During a typical treatment with a flat-top intensity profile, the radiation intensity in the epidermis is approximately constant across the outer skin location 23. However, as the temperature rises, heat diffuses away from the outer skin location into the surrounding tissue - in other words, the surrounding tissues act as a heat sink. The invention is based on the insight that the heat in the centre of the outer skin location cannot diffuse because it is surrounded by heated tissue - this leads to a hot-spot in the centre of the outer skin location, even when using a flat-top intensity profile 40.

The conventional solution to the hotspot problem is to reduce the maximum intensity 50 in the treatment beam profile until the epidermal hotspot no longer occurs. However, this also reduces the power of the laser treatment beam 22, resulting in a lower efficacy and/or extended treatment times.

Figure 6a depicts two examples of laser intensity profiles according to the invention. The axes 61, 62 and the intensity threshold 50 are the same as depicted for Figure 5a. Figure 6a depicts modified versions of the profiles depicted in Figure 5a - a modified Gaussian intensity profile 130 and a modified flat-top intensity profile 140. The modified flat-top profile 140 is the same as that 40 depicted in Figure 5a, except that the intensity at the centre of the beam cross-section is reduced to zero. The modified Gaussian profile 130 is the same as that 30 depicted in Figure 5a, except that the intensity at the centre of the beam cross-section is reduced to zero. In both cases, a central beam region 22c is provided having a very low intensity. The power in the modified intensity profiles 130, 140 is kept the same as in the intensity profiles of Figure 5a by increasing the intensity above the intensity threshold 50.

When either of the laser beam profiles of Figure 6a is used to treat skin 15, the modified epidermal 16 temperature profile 145 depicted in Figure 6b results. In Figure 6b, the axes 65, 66 and the epidermal temperature threshold 55 are the same as depicted for Figure 5b. The modified epidermal temperature profile 145 again resembles a bell-shape - however, the temperature at the centre of the outer skin location 23c is lower than the temperature at the shoulders 23a, 23b of the bell due to the presence of the additional heat sink at the center of the outer skin location 23c - no point on the temperature profile exceeds the epidermal temperature threshold 55.

The invention therefore enables hot spots in the outer skin location to be avoided without significantly reducing the power of the laser treatment beam 22 by providing a central heat sink 23c. As a result, also the power reaching the treatment location is not significantly reduced. The dimensions of the heat sink are predetermined and/or controlled to limit the maximum temperature in the outer skin location 23a, 23b, 23c during treatment. If the dimensions of the heat sink are only predetermined, a simulation of skin may be used to determine appropriate settings, depending on the desired treatment. If the dimensions of the heat sink are only controlled, some form of feedback is desired, such as the temperature sensors depicted in US patent 6015404 by Altshuler and Erofeev. Preferably, both predetermination aspects and control aspects are used to provide the most accurate temperature control in the outer skin location 23a, 23b, 23c.

The dimensions of the central heat sink required to prevent thermal damage depend on at least one parameter, including:
- the laser treatment beam intensity in the third 23c skin region,
- the separation of the first 23a and the second 23b skin region,
- the laser treatment beam intensity in the first 23a and the second 23b skin region,
- the pulse duration of the laser treatment beam 22,
- the pulse delay between treatment pulses,
- the optical and thermal properties, such as the thermal relaxation time, of the outer skin location 23a, 23b, 23c,
- the optical and thermal properties of the skin tissues surrounding the outer skin location 23a, 23b,
- the temperature of the skin tissues surrounding the outer skin location 23a, 23b,
- the wavelength spectrum, irradiance and spot size of the laser treatment beam 22,
- the depth of the outer skin location 23a, 23b, 23c below the surface of the skin 15,
- the location of the skin 15 on the body and the individual being treated, and
- the phase of the treatment cycle.

The parameters of the device 10 which may be used to predetermine and/or control the dimensions of the central heat sink include:
- the wavelength spectrum, irradiance and spot size of the laser treatment beam 22,
- the laser treatment beam intensity provided in the beam regions 22a, 22b, 22c of a transverse cross-section of the laser treatment beam 22,
- the numerical aperture (NA) of the device 10,
- the dimensions and extent of the beam regions 22a, 22b, 22c of a transverse cross-section of the laser treatment beam 22,
- the pulse duration of the laser treatment beam 22, and
- the pulse delay between treatment pulses.

The skilled person will be able to determine the parameters of the device 10 by appropriate simulation and/or measurements.

Some examples of approximate settings are:
- the deliverable energy level in the laser beam pulse may be between 0.1 and 20 mJ, measured at the surface of the skin.
- the wavelength of the light may be between 800 and 1500 nm. In this range, transmission through the skin is high and scattering and linear absorption are low.
- a diameter of the lower intensity beam region 22c in the transverse cross-section of 100-200 microns.

Many measurements have been made relating to the thermal relaxation time of skin - for example, the article "Analysis of Thermal Relaxation During Laser Irradiation of Tissue", Lasers in Surgery and Medicine 29:351-359 (2001), by Choi and Welch. In this article, thermal relaxation times were investigated using both conventional Gaussian and conventional flat-top laser beam profiles. In particular, this article analyses thermal relaxation when the skin is treated with a plurality of pulses.

It may be advantageous if the central skin heat-sink region is dimensioned such that a cross-sectional extent of the central heat sink is equal to or greater than the thermal penetration depth of the outer skin layer 16. The cross-sectional extent may be determined by measuring the distance through a plane approximately parallel to the surface of the skin 15, between opposing edges of the first 23a and the second 23b skin region. The edge of each skin region 23a, 23b may be considered to be a contour joining points whose intensity is equal to half the maximum intensity within the skin region. The thermal penetration depth, measured in microns, is typically 50-200 microns.

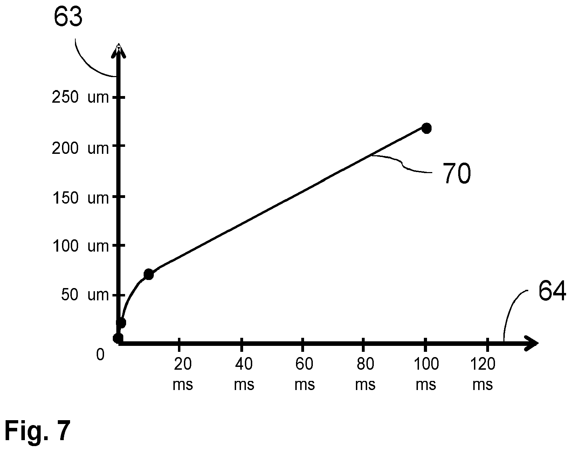

Figure 7 depicts the results of a simulation to determine the relationship 70 between thermal penetration depth and pulse duration for the upper layer of the skin. The horizontal axis 64 depicts the pulse duration in milliseconds (ms) from 0 to 120 ms in steps of 20 ms; and the vertical axis 63 depicts the thermal penetration depth in microns (µm) from 0 to 250 µm in steps of 50 µm. The relationship 70 starts at 0 ms / 4.5 µm, and then passes through 1.5 ms / 23 µm; 10 ms / 72 µm and 92ms / 225 µm. So, the thermal penetration depth increases with pulse duration.

According to the invention, the pulses of the laser treatment beam have a pulse duration which is longer than the thermal relaxation time of the outer skin region. Such relatively long pulses are conventionally avoided because pulses longer than the thermal relaxation time may result in a steady increase in temperature during treatment, which can cause the temperature of the skin to exceed the threshold for thermal damage. The thermal relaxation time for the epidermis depends on the individual, the place on the body and the parameters of the laser treatment, but is typically from 10 to 30 ms. See the "Intense Pulsed Light as a Nonablative Approach to Photoaging" article on pages 1180 to 1181 under "Pulse Durations".

So, using the relationship 70 depicted in Figure 7, a pulse length of 30 ms will result in a thermal penetration depth of about 100 micron (µm). The cross-sectional extent of the central heat sink should therefore be at least 100 micron. To achieve this, the third beam region 22c in the transverse cross-section of the laser treatment beam 22 should be approximately 100-200 microns in diameter.

It may also be advantageous to use the invention together with other measures known in the art, such as epidermal cooling.

Beam regions of non-zero and lower intensity within the transverse beam cross-section may be provided by configuring and arranging the laser light source 20 to operate in a higher order mode with a central region having a substantially lower intensity than at the periphery, i.e. not in the fundamental mode and not in a mixed multi-mode where the output is a combination of a plurality of higher-order modes. The higher order mode should be pure enough to create the required regions of non-zero and substantially lower intensity. Any methods known in the art may be used to achieve this, such as appropriate configuration of the laser cavity or resonator. For example, the article "Generation of pure TEMpO modes using a friendly intra-cavity laser beam shaping technique" by Cagniot, Fromager et al; Laser Beam Shaping XII; SPIE Vol. 8130, 813006 (2011) discloses a model for generating higher modes with phase and amplitude DOEs in a laser cavity (resonator). For example, a pi-phase plate is inserted into a plano-concave cavity.

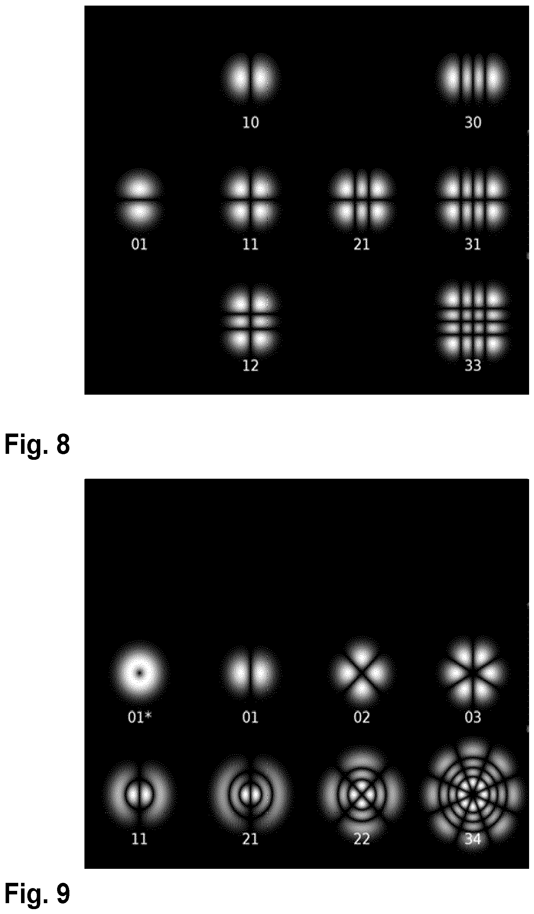

Fig. 9 depicts examples of known Laguerre-Gaussian modes that may be used, such as LG-01, LG-02 and LG-03. In this patent application, the standard notation for LG modes is used - the first index indicates the number of radial mode orders (p), and the second index indicates the number of angular mode orders (1).

The intensity regions of the invention may also be provided by configuring and arranging the laser light source 20 to operate in the LG-01* mode, where p=0 and 1=1. The "*" indicates that this is the so-called "doughnut" or annular mode depicted in Figure 3. The first 22a and the second 22b beam region are contiguous, forming part of an annular region of non-zero light intensity. The third beam region 22c is configured to form a central region of lower light intensity, and said central region of lower intensity is enclosed by the annular region of non-zero light intensity.

LG modes such as LG-11, LG-21, LG-22 and LG-34 may also be used to provide a plurality of suitable regions of low intensity 22c.

Fig. 8 depicts examples of known Hermite-Gaussian modes that may be used, such as HG-01, HG-10 or HG-11. Figure 4 depicts a transverse cross-section 122 approximating the HG-01 mode - two non-contiguous regions 122a, 122b of non-zero intensity are provided, which are separated along the total length of their borders within the transverse cross-section of the laser treatment beam 122 by the third region 122c of lower intensity. This intensity profile may be created by appropriate modifications to the laser source, or by disposing a suitable mask in the treatment beam 22 at an appropriate position. HG modes such as HG-12, HG-21, HG-30, HG-31 and HG-33 may also be used to provide a plurality of suitable regions 22c of low intensity.

More details on intensity distributions in higher-order laser modes can be found in Chapter 11: Laser Beam Diagnostics in a Spatial Domain by Tae Moon Jeong and Jongmin Lee, in the book Laser Pulse Phenomena and Applications, edited by F. J. Duarte, ISBN 978-953-1007-405-4.

It will be clear to the skilled person that suitable intensity profiles may also be created by a combination of modifications and/or optical elements within the laser cavity, and optical elements disposed along the optical path outside the laser cavity.

The device and method according to the invention may be used for any suitable treatment of the skin, in particular non-invasive wrinkle reduction in the skin, actinic keratosis, scar tissue or acne and reduction of pigmentation spots. The device and method may be used for selective photothermolysis, in particular for ablative and non-ablative techniques.

The laser light source 20 may be disposed outside of the device 10, and connected using an optical fiber. This provides a small and lightweight applicator unit, with the bulkier and heavier laser source etc., in a separate and stationary unit.

It should be noted that the above-mentioned embodiments illustrate rather than limit the invention, and that those skilled in the art will be able to design many alternative embodiments.

In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

### Reference numbers

10 Skin treatment device
12 Optical system
13 Device optical axis
15 Skin
16 Epidermis layer of skin
17 Dermis layer of skin
20 Light source for generating laser treatment beam (22)
22 Laser treatment beam
22a First beam region of non-zero light intensity
22b Second beam region of non-zero light intensity
22c Third beam region of lower light intensity than the non-zero light intensity

23a First skin region in the outer skin layer
23b Second skin region in the outer skin layer
23c Third skin region in the outer skin
25 Focal spot
30 Gaussian laser beam intensity profile
35 Epidermal temperature profile when exposed to Gaussian intensity profile
40 Flat-top laser beam intensity profile
45 Epidermal temperature profile when exposed to flat-top intensity profile
50 Intensity threshold
55 Epidermal temperature threshold
61 Intensity axis (vertical)
62 Position in laser beam transverse cross-section axis (horizontal)
63 Thermal penetration depth in microns (vertical axis)
64 Pulse duration in milliseconds (horizontal axis)
65 Epidermal temperature axis (vertical)
66 Position in outer skin location transverse cross-section axis (horizontal)
70 Relationship between thermal penetration depth in skin and pulse duration
122 Second embodiment of treatment laser beam
122a (alternative): 1st beam region = non-zero intensity
122b (alternative): 2nd beam region = non-zero intensity
122c (alternative): 3^{rd} beam region of lower light intensity than the non-zero light intensity
130 Modified Gaussian laser beam intensity profile
140 Modified flat-top laser beam intensity profile
145 Modified epidermal temperature profile

## Claims

1. A non-invasive device (10) for treatment of an inner skin layer (17) using laser light, the device (10) comprising:
- a light source (20) and an associated optical system (12) for generating pulses of a laser treatment beam (22) along a treatment optical axis (13) of the device (10), the light source (20) and the optical system (12) being configured and arranged to provide, within a transverse cross-section of the laser treatment beam (22) perpendicular to the treatment optical axis (13):
- a first (22a,122a) and a second (22b,122b) beam region of non-zero light intensity disposed at a periphery of the transverse cross-section, and
- a third beam region (22c,122c), arranged between the first (22a,122a) and the second (22b,122b) beam region, of lower light intensity than said non-zero light intensity;
the optical system (12) being further configured and arranged:
- to focus, in use, the treatment laser beam (22) to a focal spot (25) in the inner skin layer (17) disposed on the treatment optical axis (13);
- to guide, in use, the laser treatment beam (22) such that the laser treatment beam (22) with the first (22a,122a) and the second (22b,122b) beam region passes along the treatment optical axis (13) through an outer skin layer (16) to the focal spot (25), whereby the first (22a,122a) and the second (22b,122b) beam region pass through, respectively, a first (23a) and a second (23b) skin region in the outer skin layer (16);
wherein an extent of the third beam region (22c,122c) is predetermined and/or controlled to provide a corresponding third skin region (23c) in the outer skin layer (16), arranged between the first (23a) and the second skin region (23b),
wherein the lower light intensity of the third beam region is predetermined and/or controlled to provide a maximum temperature during a pulse of the laser treatment beam within the third skin region (23c) which is lower than maximum temperatures during said pulse of the laser treatment beam within the first (23a) and the second (23b) skin regions;
wherein the light source (20) is configured and arranged to provide, in use, a pulse duration of the pulse of the laser treatment beam which is longer than a thermal relaxation time of the first (23 a) and the second (23b) skin region; and
**characterized in that** the optical
system (12) is configured and arranged to provide the laser treatment beam (22) that is to impinge on a surface of the outer skin layer (17) having a diameter that is approximately the same as the diameter of the laser treatment beam (22) generated by the light source (20).

2. The device according to claim 1, wherein the light source (20) or optical system (12) comprises at least one optical element, configured and arranged to provide the first, the second and the third beam region, and wherein the optical element is a central mask, a ring aperture, a Spatial Light Modulator (SLM), a Diffractive-Optical element (DOE), a phase mask, a spiral waveplate, a vortex waveplate, a Pitch-Fork Hologram, a Q-Plate, or a Cylindrical Mode Converter.

3. The device according to claim 1, wherein the optical system (12) is further configured and arranged to focus, in use, the treatment laser beam (22) to a focal spot (25) disposed in the dermis (17), and to guide, in use, the first (22a,122a) and the second (22b,122b) beam region along the treatment optical axis (13) through the outer skin layer (16) to the focal spot (25), whereby the first (23a) and the second (23b) skin region are disposed in the stratum corneum, epidermis, or skin surface layer.

4. The device according to claim 1, wherein the light source (20) is configured and arranged to provide, in use, a pulse duration of the pulse of the laser treatment beam which is longer than a thermal relaxation time of the outer skin layer (16) and shorter than a thermal relaxation time of the inner skin layer (17).

5. The device according to claim 1, wherein the first (22a,122a), the second (22b,122b) and the third (22c,122c) beam region are configured to provide a third skin region (23c) having a cross-sectional extent greater than or equal to a thermal penetration depth of the outer skin layer (16) at said pulse duration.

6. The device according to claim 1, wherein the first (22a) and the second (22b) beam region are contiguous and form part of an annular region of non-zero light intensity, the third beam region (22c) being configured to form a central region of lower light intensity, and wherein the central region of lower light intensity is enclosed by the annular region of non-zero light intensity.

7. The device according to claim 1, wherein the first (122a) and the second (122b) beam region are non-contiguous, and the third beam region (122c) is configured so as to separate the first (122a) and the second (122b) beam region throughout the length of their borders within the transverse cross-section of the laser treatment beam (122).

8. A non-invasive, non-surgical and non-therapeutic method of treating an inner skin layer (17) using a device (10) for generating laser light, the device (10) comprising a light source (20) and an associated optical system (12) for generating pulses of a laser treatment beam (22) along a treatment optical axis (13) of the device (10), the method comprising:
- generating the laser treatment beam (22) with a transverse cross-section perpendicular to the treatment optical axis (13) comprising:
- a first (22a) and a second (22b) beam region of non-zero light intensity disposed at a periphery of the transverse cross-section, and
- a third beam region (22c), arranged between the first (22a) and the second (22b) beam region, of lower light intensity than said non-zero light intensity;
- focusing the treatment laser beam (22) to a focal spot (25) in the inner skin layer (17), disposed on the treatment optical axis (13), using the optical system (12);
- guiding the laser treatment beam (22) such that the laser treatment beam (22) with the first (22a) and the second (22b) beam region passes through an outer skin layer (16) along the treatment optical axis (13) to the focal spot (25), whereby the first (22a) and the second (22b) beam region pass through, respectively, a first (23a) and a second (23b) skin region in the outer skin layer (16);
- predetermining and/or controlling an extent of the third beam region (22c) to provide a corresponding third skin region (23c) in the outer skin layer (16), arranged between the first (23a) and the second (23b) skin region,
- predetermining and/or controlling the lower light intensity of the third beam region to provide a maximum temperature during a pulse of the laser treatment beam within the third skin region (23c) which is lower than maximum temperatures during said pulse of the laser treatment beam within the first (23a) and the second (23b) skin region; and
- configuring and arranging the light source (20) to provide, in use, a pulse duration of the pulse of the laser treatment beam which is longer than a thermal relaxation time of the first (23a) and the second (23b) skin region; and
**characterized by** configuring and arranging the optical system (12) to provide wherein the laser treatment beam (22) that impinges on a surface of the outer skin layer (17) having a diameter that is approximately the same as the diameter of the laser treatment beam (22) generated by the light source (20).

## Patentansprüche

1. Vorrichtung (10) zur nichtinvasiven Behandlung einer inneren Hautschicht (17) unter Verwendung von Laserlicht, wobei die Vorrichtung (10) umfasst:
- eine Lichtquelle (20) und ein zugehöriges optisches System (12) zum Erzeugen von Pulsen eines Laserbehandlungsstrahls (22) entlang einer optischen Behandlungsachse (13) der Vorrichtung (10), wobei die Lichtquelle (20) und das optische System (12) konfiguriert und eingerichtet sind, um innerhalb eines Querschnitts des Laserbehandlungsstrahls (22) senkrecht zu der optischen Behandlungsachse (13) Folgendes bereitzustellen:
- eine erste (22a, 122a) und eine zweite (22b, 122b) Strahlregion einer Lichtintensität ungleich null, die an einer Peripherie des Querschnitts angeordnet ist, und
- eine dritte (22c, 122c) Strahlregion, die zwischen der ersten (22a, 122a) und der zweiten (22b, 122b) Strahlregion eingerichtet ist, von geringerer Lichtintensität als die Lichtintensität ungleich null;
wobei das optische System (12) weiter konfiguriert und eingerichtet ist:
- um im Betrieb den Laserbehandlungsstrahl (22) auf einen Brennfleck (25) in der inneren Hautschicht (17) zu fokussieren, der auf der optischen Behandlungsachse (13) angeordnet ist;
- um im Betrieb den Laserbehandlungsstrahl (22) zu führen, sodass der Laserbehandlungsstrahl (22) mit der ersten (22a, 122a) und der zweiten (22b, 122b) Strahlregion entlang der optischen Behandlungsachse (13) durch eine äußere Hautschicht (16) zu dem Brennfleck (25) verläuft, wodurch die erste (22a, 122a) und die zweite (22b, 122b) Strahlregion jeweils durch eine erste (23a) und eine zweite (23b) Hautregion in der äußeren Hautschicht (16) verlaufen;
wobei eine Ausdehnung der dritten Strahlregion (22c, 122c) vorbestimmt und/ oder gesteuert ist, um eine entsprechende dritte Hautregion (23c) in der äußeren Hautschicht (16) bereitzustellen, die zwischen der ersten (23a) und der zweiten (23b) Hautregion angeordnet ist,
wobei die geringere Lichtintensität der dritten Strahlregion vorbestimmt und/ oder gesteuert ist, um eine maximale Temperatur während eines Pulses des Laserbehandlungsstrahls innerhalb der dritten Hautregion (23c) bereitzustellen, die geringer als die maximalen Temperaturen während des Pulses des Laserbehandlungsstrahls innerhalb der ersten (23a) und der zweiten (23b) Hautregion ist;
wobei die Lichtquelle (20) konfiguriert und eingerichtet ist, um im Betrieb eine Pulsdauer des Pulses des Laserbehandlungsstrahls bereitzustellen, die länger als eine thermische Relaxationszeit der ersten (23a) und der zweiten (23b) Hautregion ist; und
**dadurch gekennzeichnet, dass** das optische System (12) konfiguriert und eingerichtet ist, um den Laserbehandlungsstrahl (22) bereitzustellen, der zum Beaufschlagen einer Oberfläche der äußeren Hautschicht (17) ist, der einen Durchmesser aufweist, der annähernd derselbe ist, wie der Durchmesser des Laserbehandlungsstrahls (22), der durch die Lichtquelle (20) erzeugt wird.

2. Vorrichtung nach Anspruch 1, wobei die Lichtquelle (20) oder das optische System (12) mindestens ein optisches Element umfasst, konfiguriert und eingerichtet, um die erste, die zweite, und die dritte Strahlregion bereitzustellen, und wobei das optische Element eine zentrale Maske, eine Ringöffnung, ein räumlicher Lichtmodulator (SLM), ein lichtbrechendes optisches Element (DOE), eine Phasenmaske, eine Spiralwellenplatte, eine Wirbelwellenplatte, ein Pitch-Fork Hologramm, eine Q-Platte oder ein zylindrischer Moden-Wandler ist.

3. Vorrichtung nach Anspruch 1, wobei das optische System (12) weiter konfiguriert und eingerichtet ist, um im Betrieb den Laserbehandlungsstrahl (22) auf einen Brennfleck (25) zu fokussieren, der in der Dermis (17) angeordnet ist, und im Betrieb die erste (22a, 122a) und die zweite (22b, 122b) Strahlregion entlang der optischen Behandlungsachse (13) durch die äußere Hautschicht (16) zu dem Brennfleck (25) zu führen, wodurch die erste (23a) und die zweite (23b) Hautregion in der Hornschicht, Epidermis oder Hautoberflächenschicht angeordnet sind.

4. Vorrichtung nach Anspruch 1, wobei die Lichtquelle (20) konfiguriert und eingerichtet ist, um im Betrieb eine Pulsdauer des Pulses des Laserbehandlungsstrahls bereitzustellen, die länger als eine thermische Relaxationszeit der äußeren Hautschicht (16) und kürzer als eine thermische Relaxationszeit der inneren Hautschicht (17) ist.

5. Vorrichtung nach Anspruch 1, wobei die erste (22a, 122a), die zweite (22b, 122b) und die dritte (22c, 122c) Strahlregion konfiguriert sind, um eine dritte Hautregion (23c) bereitzustellen, die eine Querschnittausdehnung größer als oder gleich eine thermische Eindringtiefe der äußeren Hautschicht (16) auf der Pulsdauer aufweist.

6. Vorrichtung nach Anspruch 1, wobei die erste (22a) und die zweite (22b) Strahlregion fortlaufend sind und einen Teil einer ringförmigen Region einer Lichtintensität ungleich null bilden, wobei die dritte Strahlregion (22c) konfiguriert ist, um eine zentrale Region von geringerer Lichtintensität zu bilden, und wobei die zentrale Region von geringerer Lichtintensität durch die ringförmige Region Lichtintensität ungleich null eingeschlossen ist.

7. Vorrichtung nach Anspruch 1, wobei die erste (122a) und die zweite (122b) Strahlregion nicht fortlaufend sind, und die dritte Strahlregion (122c) konfiguriert ist, um die erste (122a) und die zweite (122b) Strahlregion über eine Länge ihrer Grenzen innerhalb des Querschnitts des Laserbehandlungsstrahls (122) zu trennen.

8. Verfahren zur nichtinvasiven, nicht chirurgischen und nicht therapeutischen Behandlung einer inneren Hautschicht (17) unter Verwendung einer Vorrichtung (10) zum Erzeugen von Laserlicht, wobei die Vorrichtung (10) eine Lichtquelle (20) und ein zugehöriges optisches System (12) zum Erzeugen von Pulsen eines Laserbehandlungsstrahls (22) entlang einer optischen Behandlungsachse (13) der Vorrichtung (10) umfasst, wobei das Verfahren umfasst:
- Erzeugen des Laserbehandlungsstrahls (22) mit einem Querschnitt senkrecht zu der optischen Behandlungsachse (13) Folgendes umfassend:
- eine erste (22a) und eine zweite (22b) Strahlregion einer Lichtintensität ungleich null, die an einer Peripherie des Querschnitts angeordnet ist, und
- eine dritte (22c) Strahlregion, die zwischen der ersten (22a) und der zweiten (22b) Strahlregion eingerichtet ist, von geringerer Lichtintensität als die Lichtintensität ungleich null;
- Fokussieren des Laserbehandlungsstrahls (22) auf einen Brennfleck (25) in der inneren Hautschicht (17), der auf der optischen Behandlungsachse (13) angeordnet ist, unter Verwendung des optischen Systems (12);
- Führen des Laserbehandlungsstrahls (22), sodass der Laserbehandlungsstrahl (22) mit der ersten (22a) und der zweiten (22b) Strahlregion entlang der optischen Behandlungsachse (13) durch eine äußere Hautschicht (16) zu dem Brennfleck (25) verläuft, wodurch die erste (22a) und die zweite (22b) Strahlregion jeweils durch eine erste (23a) und eine zweite (23b) Hautregion in der äußeren Hautschicht (16) verlaufen;
Vorbestimmen und/ oder Steuern einer Ausdehnung der dritten Strahlregion (22c), um eine entsprechende dritte Hautregion (23c) in der äußeren Hautschicht (16) bereitzustellen, die zwischen der ersten (23a) und der zweiten (23b) Hautregion angeordnet ist,
Vorbestimmen und/ oder Steuern der geringeren Lichtintensität der dritten Strahlregion, um eine maximale Temperatur während eines Pulses des Laserbehandlungsstrahls innerhalb der dritten Hautregion (23c) bereitzustellen, die geringer als die maximalen Temperaturen während des Pulses des Laserbehandlungsstrahls innerhalb der ersten (23a) und der zweiten (23b) Hautregion ist;
Konfigurieren und Einrichten der Lichtquelle (20), um im Betrieb eine Pulsdauer des Pulses des Laserbehandlungsstrahls bereitzustellen, die länger als eine thermische Relaxationszeit der ersten (23a) und der zweiten (23b) Hautregion ist; und
**gekennzeichnet durch** das Konfigurieren und Einrichten des optischen Systems (12), um den Laserbehandlungsstrahl (22) bereitzustellen, der eine Oberfläche der äußeren Hautschicht (17) beaufschlagt, der einen Durchmesser aufweist, der annähernd derselbe ist, wie der Durchmesser des Laserbehandlungsstrahls (22), der durch die Lichtquelle (20) erzeugt wird.

## Revendications

1. Dispositif non invasif (10) pour le traitement d'une couche de peau interne (17) en utilisant une lumière laser, le dispositif (10) comprenant :
- une source de lumière (20) et un système optique associé (12) pour générer des impulsions d'un faisceau de traitement laser (22) le long d'un axe optique de traitement (13) du dispositif (10), la source de lumière (20) et le système optique (12) étant configurés et agencés pour fournir, dans une section transversale du faisceau de traitement laser (22) perpendiculaire à l'axe optique de traitement (13) :
- une première (22a, 122a) et une deuxième (22b, 122b) région de faisceau d'intensité lumineuse non nulle disposées à une périphérie de la section transversale, et
- une troisième région de faisceau (22c, 122c), agencée entre la première (22a, 122a) et la deuxième région de faisceau (22b, 122b), d'une intensité lumineuse inférieure à celle de l'intensité lumineuse non nulle ;
le système optique (12) étant en outre configuré et agencé :
- pour focaliser, en cours d'utilisation, le faisceau de traitement laser (22) sur un point focal (25) dans la couche de peau interne (17) disposée sur l'axe optique de traitement (13) ;
- pour guider, en cours d'utilisation, le faisceau de traitement laser (22) de sorte que le faisceau de traitement laser (22) avec les première (22a, 122a) et deuxième (22b, 122b) zones de faisceau passe le long de l'axe optique de traitement (13) à travers une couche de peau externe (16) jusqu'au point focal (25), de sorte que la première (22a, 122a) et la deuxième (22b, 122b) zones de faisceau passent à travers, respectivement, une première (23a) et une deuxième (23b) région de peau dans la couche de peau externe (16) ;
dans lequel une étendue de la troisième région de faisceau (22c, 122c) est prédéterminée et/ou commandée pour fournir une troisième région de peau correspondante (23c) dans la couche de peau externe (16), agencée entre la première (23a) et la deuxième région de peau (23b),
dans lequel l'intensité lumineuse inférieure de la troisième région de faisceau est prédéterminée et/ou commandée pour fournir une température maximum pendant une impulsion du faisceau de traitement laser dans la troisième région de peau (23c) qui est inférieure aux températures maximums pendant ladite impulsion du faisceau de traitement laser dans la première (23a) et la deuxième (23b) région de peau ;
dans lequel la source de lumière (20) est configurée et agencée pour fournir, en utilisation, une durée d'impulsion de l'impulsion du faisceau de traitement laser qui est supérieure à un temps de relaxation thermique de la première (23a) et la deuxième (23b) région de peau ; et
**caractérisé en ce que** le système optique (12) est configuré et agencé pour fournir le faisceau de traitement laser (22) qui doit frapper une surface de la couche de peau externe (17) ayant un diamètre approximativement identique au diamètre du faisceau de traitement laser (22) généré par la source de lumière (20).

2. Dispositif selon la revendication 1, dans lequel la source de lumière (20) ou le système optique (12) comprend au moins un élément optique, configuré et agencé pour fournir la première, la deuxième et la troisième région de faisceau, et dans lequel l'élément optique est un masque central, une ouverture annulaire, un modulateur de lumière spatiale (SLM), un élément optique diffractif (DOE), un masque de phase, une lame d'onde en spirale, une lame d'onde de vortex, un hologramme de diapason, un plateau en forme de « Q », ou un convertisseur de mode cylindrique.

3. Dispositif selon la revendication 1, dans lequel le système optique (12) est en outre configuré et agencé pour focaliser, en cours d'utilisation, le faisceau de traitement laser (22) sur un point focal (25) disposé dans le derme (17), et pour guider, en cours d'utilisation, la première (22a, 122a) et la deuxième (22b, 122b) région de faisceau le long de l'axe optique de traitement (13) à travers la couche de peau externe (16) jusqu'au point focal (25), de sorte que la première (23a) et la deuxième (23b) région de peau sont disposées dans la couche de cornée, l'épiderme ou la couche de surface de peau.

4. Dispositif selon la revendication 1, dans lequel la source de lumière (20) est configurée et agencée pour fournir, en utilisation, une durée d'impulsion de l'impulsion du faisceau de traitement laser qui est plus longue qu'un temps de relaxation thermique de la couche de peau externe (16) et plus court qu'un temps de relaxation thermique de la couche de peau interne (17).

5. Dispositif selon la revendication 1, dans lequel la première (22a, 122a), la deuxième (22b, 122b) et la troisième (22c, 122c) région de faisceau sont configurées pour fournir une troisième région de peau (23c) ayant une étendue de section transversale supérieure ou égale à une profondeur de pénétration thermique de la couche de peau externe (16) à ladite durée d'impulsion.

6. Dispositif selon la revendication 1, dans lequel les première (22a) et deuxième (22b) région de faisceau sont contiguës et forment une partie d'une région annulaire d'intensité lumineuse non nulle, la troisième région de faisceau (22c) étant configurée pour former une région centrale d'intensité lumineuse plus faible, et
dans lequel la région centrale d'intensité lumineuse inférieure est entourée par la région annulaire d'intensité lumineuse non nulle.

7. Dispositif selon la revendication 1, dans lequel les première (122a) et deuxième (122b) région de faisceau ne sont pas contiguës, et la troisième région de faisceau (122c) est configurée de manière à séparer la première (122a) et la deuxième (122b) région de faisceau sur toute la longueur de leurs bordures dans la section transversale du faisceau de traitement laser (122).

8. Procédé de traitement d'une couche de peau interne (17) non invasif, non chirurgical et non thérapeutique, en utilisant un dispositif (10) pour générer une lumière laser, le dispositif (10) comprenant une source de lumière (20) et un système optique (12) associé pour générer des impulsions d'un faisceau de traitement laser (22) le long d'un axe optique de traitement (13) du dispositif (10), le procédé comprenant les étapes consistant à :
- générer le faisceau de traitement laser (22) avec une section transversale perpendiculaire à l'axe optique de traitement (13) comprenant :
- une première (22a) et une deuxième (22b) région de faisceau d'intensité lumineuse non nulle disposées à une périphérie de la section transversale, et
- une troisième région de faisceau (22c), agencée entre la première (22a) et la deuxième (22b) région de faisceau, d'une intensité lumineuse inférieure à ladite intensité lumineuse non nulle ;
- focaliser le faisceau de traitement laser (22) sur un point focal (25) dans la couche de peau interne (17), disposée sur l'axe optique de traitement (13), en utilisant le système optique (12) ;
- guider le faisceau de traitement laser (22) de telle sorte que le faisceau de traitement laser (22) avec la première (22a) et la deuxième (22b) région de faisceau passe à travers une couche de peau externe (16) le long de l'axe optique de traitement (13) jusqu'au point focal (25), de sorte que la première (22a) et la deuxième (22b) région de faisceau passent à travers, respectivement, une première (23a) et une deuxième (23b) région de peau dans la couche de peau externe (16) ;
- prédéterminer et/ou commander une étendue de la troisième région de faisceau (22c) pour fournir une troisième région de peau correspondante (23c) dans la couche de peau externe (16), agencée entre la première (23a) et la deuxième (23b) région de peau,
- prédéterminer et/ou commander l'intensité lumineuse inférieure de la troisième région de faisceau pour fournir une température maximum pendant une impulsion du faisceau de traitement laser dans la troisième région de peau (23c) qui est inférieure aux températures maximums pendant ladite impulsion du faisceau de traitement laser à l'intérieur de la première (23a) et de la deuxième (23b) région de peau ; et
- configurer et agencer la source de lumière (20) pour fournir, en cours d'utilisation, une durée d'impulsion du faisceau de traitement laser qui est supérieure à un temps de relaxation thermique de la première (23a) et de la deuxième (23b) région de peau ; et
**caractérisé par** la configuration et l'agencement du système optique (12) pour fournir
dans lequel le faisceau de traitement laser (22) qui frappe une surface de la couche de peau externe (17) ayant un diamètre qui est approximativement identique au diamètre du faisceau de traitement laser (22) généré par la source de lumière (20).
